# EUROPEAN PATENT SPECIFICATION

(11) **EP 3 428 117 B1**
(45) Date of publication and mention of the grant of the patent: **27.07.2022**
(21) Application number: 17762582.9
(22) Date of filing: 02.03.2017
(51) Int. Cl.: B82Y 5/00, A61L 15/26, A61L 15/40, A61L 27/18, A61L 27/36, A61L 27/54

(54) **HYBRID ALOE VERA NANOFIBRES**
HYBRIDE ALOE-VERA-NANOFASERN
NANOFIBRES HYDRIDES D'ALOE VERA

(30) Priority: 08.03.2016 ES 201600173
(43) Date of publication of application: 16.01.2019
(60) Divisional application: 18173213.2
(73) Proprietor: Universidad de Las Palmas de Gran Canaria, 35001 Las Palmas de Gran Canaria (ES)
(72) Inventor: MONZÓN MAYOR, Maximina, 35001 - Islas Canarias (ES); ROMERO ALEMÁN, Maria del Mar, 35001 - Islas Canarias (ES); HERNÁNDEZ RODRÍGUEZ, José Enrique, 35016, Las Palmas (ES); PÉREZ GALVÁN, José Manuel, 35016, Las Palmas (ES)
(74) Representative: ABG Intellectual Property Law, S.L.
(86) International application number: PCT/ES2017/000029
(87) International publication number: WO 2017/153619

(56) References cited:
- WO-A1-2013/035072
- WO-A1-2014/027965
- WO-A1-2014/142675
- WO-A1-2015/157485
- WO-A2-2009/133059
- SUGANYA S ET AL.: 'Naturally derived biofunctional nanofibrous scaffold for skin tissue regeneration' INTERNATIONAL JOURNAL OF BIOLOGICAL MACROMOLECULES, [Online] vol. 68, July 2014, ISSN 0141-8130 pages 135 - 143, XP028849087 ISSN: 0141-8130 Retrieved from the Internet: <URL:https://doi.org/10.1016/j.ijbiomac.201 4.04.031>
- PRABHAKARAN MOLAMMA P ET AL.: 'Electrospun Aligned PHBV/Collagen Nanofibers as Substrates for Nerve Tissue Engineering' BIOTECHNOLOGY AND BIOENGINEERING, [Online] vol. 110, no. 10, October 2013, ISSN 0006-3592 pages 2775 - 2784, XP055421082 ISSN: 0006-3592 Retrieved from the Internet: <URL:doi: 10.1002/bit.24937>

## Description

### Field of the Invention

The present invention belongs to the field of tissue engineering. In particular, it relates to the application of hybrid Aloe vera nanofibers to nerve regeneration.

### Background of the Invention

Nerve tissue controls the homeostasis of all organs and systems of the body. Proper nerve tissue regeneration contributes to the functional regeneration of other tissues forming different organs. After traumatic injuries, successful axonal regrowth both in the central nervous system (CNS) and in large gaps between peripheral nerve endings and functional target tissue reinnervation as a result of injuries in the CNS, peripheral nerves, or local wounds (e.g., skin wounds) constitutes a challenge in the field of regenerative biomedicine today. Peripheral nerves present spontaneous regrowth capacity provided that contact between the nerve endings is restored since the distal nerve ending Schwann cells provide a favorable microenvironment. However, the structure and function of regenerated nerves differ from normal health conditions. Furthermore, target organ reinnervation is usually clinically disappointing, with significant and persistent functional deficits.

Up until now, the main method for peripheral nerve regeneration consists of replacing the damaged region with autologous and heterologous tissue transplants. However, these tissue transplants present significant limitations, such as the limited availability of autologous tissue transplants and the possibility of immune rejection of said transplants. The use of natural and synthetic materials which reproduce the natural micrometric and nanometric organization of the extracellular matrix of healthy tissues and provide an optimal microenvironment for cell adhesion, growth, proliferation, and differentiation has been proposed as an alternative.

The usefulness of synthetic polymers such as poly-3-hydroxybutyrate-co-3-hydroxyvalerate (PHBV) and poly-L-lactic acid (PLLA) in axonal regrowth is recognized in the literature [PLLA (Corey et al., J. Biomed. Mater Res. A 2007, 83(3)636-645; Wang et al., J. Neural Eng. 2009, 6(1), 016001), PHBV (Masaeli et al., 2013, PLoS One 8(2) e57157), Prabhakaran et al., 2013, Biotechnol. Bioeng.110(10)2775-84)]. Furthermore, I Uslu et al. (Hacettepe J. Biol. & Chem., 2010, 38(1)) disclose the use of hybrid Aloe vera nanofibers with synthetic polyvinyl alcohol/polyvinylpyrrolidone/polyethylene glycol polymers for wound dressing. Gupta et al (J. Biomater. Tissue Eng., 2013, 3(5) 503-11) disclose hybrid Aloe vera nanofibers, polyvinyl alcohol, polyethylene oxide, and carboxymethyl cellulose. Jithendra et al. (ACS Appl. Matter. Interfaces, 2013, 5, 7291-8) disclose collagen, chitosan, and Aloe vera nanofibers for tissue engineering. Shanmugavel et al. (J. Biomatter. Appl., 2013, 29(1) 46-58) disclose silk fibroin, caprolactone, and Aloe vera nanofibers for bone tissue engineering. Sungaya et al. 2014 (Iran Polym J., 23, 237-248) disclose silk fibroin, hydroxyapatite, and Aloe vera nanofibers for ossification. Suganya et al. (Int. J. Bioi. Macromol. 68(7), 135-143 (2014)) discloses nanofibrous scaffolds made by electrospinning a solution containing PLACL, silk fibroin and Aloe vera powder. In addition, WO2015/157485A describes a device for promoting healing at a connection site between tubular biologic structures comprises a porous construct of nanofibers spun from a biocompatible material (polycaprolactone, polylactide, or polyglycolide) in a sheet. Furthermore, Wang and Ji-Huan disclose the production of hybrid honey and polyvinyl alcohol (PVA) nanofibers (Thermal Science 2013, 17:1549-1550). Maleki *et al.* propose the use of hybrid honey and PVA nanofibers as a wound dressing (J. Appl. Polym. Sci 2013, 127:4086-4092). Arslan *et al.* describe the production of hybrid honey and polyethylene terephthalate (PET) nanofibers and the potential use thereof as a wound dressing (J. Biomater. Sci. Polym. Ed. 2014, 25(10):999-1012). Sarhan *et al.* publish the production of hybrid honey, PVA, and chitosan nanofibers for use in tissue engineering and as a wound dressing (Material Science and Engineering C 2016, 67:276-284; Applied Materials and Interfaces 2016 8:6379-6390). None of these documents mentions structures that are efficient in nerve tissue growth and regeneration.

There is therefore a need in the state of the art to attain new structures that are more efficient in nerve regeneration, particularly in functional target tissue, peripheral nerve, or local wound reinnervation.

### Description of the Invention

The object of the present invention is to provide structures which allow nerve reconnection after peripheral nerve axotomy and to improve sensory recovery from injuries (e.g., burns, ulcers, surgical incisions, etc.) in sensory organs such as the skin, among others. The hybrid nanofibers provided in the present invention promote nerve regeneration, additionally acting as a structural support for nerve tissue growth. Furthermore, the biocompatibility of the hybrid nanofibers of the invention prevents rejection by the body.

In a first aspect, the invention relates to hybrid nanofibers comprising a mixture of
i) the components of an Aloe vera gel and
ii) a synthetic polymer,
   wherein the synthetic polymer is selected from poly-3-hydroxybutyrate-co-3-hydroxyvalerate (PHBV), poly-L-lactic acid (PLLA), polydioxanone (PDS), and derived mixtures,
   wherein the average diameter of the hybrid nanofibers is comprised between 0.3 and 1.5 microns, and
   wherein the components of an Aloe vera gel are all the components of said Aloe vera gel or Aloe vera gel derivatives selected from gel lyophilisate, gel secretion, plant extract, solution of the gel, or in solution without aloin and/or with a high concentration of acemannan.

In a second aspect, the invention relates to a method for producing hybrid Aloe vera nanofibers, wherein the method comprises:
a) preparing a solution of an Aloe vera gel or of Aloe vera gel derivatives in a solvent selected from hexafluoride-2-propanol, polyvinyl alcohol (PVA), a chloroform:methanol solution, or derived mixtures,
b) mixing the solution of Aloe vera gel or of Aloe vera gel derivatives of step a) with a synthetic polymer selected from poly-3-hydroxybutyrate-co-3-hydroxyvalerate (PHBV), poly-L-lactic acid (PLLA), polydioxanone (PDS), and derived mixtures to form a hybrid polymer solution, and
c) injecting the mixture of step b) into electrospinning equipment for producing hybrid Aloe vera nanofibers by electrospinning,
wherein the Aloe vera gel derivatives in step a) are selected from gel lyophilisate, gel secretion, plant extract, solution of the gel, or in solution without aloin and/or with a high concentration of acemannan.

Likewise, the invention also relates to hybrid nanofibers comprising a mixture of the components of an Aloe vera gel and a synthetic polymer selected from poly-3-hydroxybutyrate-co-3-hydroxyvalerate (PHBV), poly-L-lactic acid (PLLA), polydioxanone (PDS), and derived mixtures, wherein the hybrid nanofibers have an average diameter comprised between 0.3 and 1.5 microns, obtained by means of the aforementioned method.

In one aspect, the invention relates to the hybrid Aloe vera nanofibers for use as a medicinal product. In another additional aspect, the invention relates to the use of the hybrid Aloe vera nanofibers for producing a medicinal product for nerve tissue regeneration in any vertebrate or for promoting the growth, proliferation, or differentiation of any cell type located in any tissue, organ, or organ system where the nerve tissue is present. In particular, the regeneration or growth of the nerve tissue in the presence of the hybrid nanofibers of the invention occurs without requiring the presence of other additives or growth factors promoting the growth of the nerve tissue.

Furthermore, the invention also relates to the use of the hybrid nanofibers for producing tubular prostheses, dressings, sutures, or surgical meshes, and to the tubular prostheses, dressings, sutures, or surgical meshes comprising the hybrid Aloe vera nanofibers.

### Description of the Drawings

The drawings included in the description illustrate particular embodiments of the present invention. Furthermore, in combination with the text of the description, the drawings are used to explain the principles on which the invention is based.
Figure 1 shows a table listing the particular conditions used for the formation of synthetic polymer nanofibers and hybrid Aloe vera nanofibers of the invention by electrospinning.
Figure 2 shows the scanning electron microscopy micrograph of electrospun polymers: A. PLLA. B. PLLA + Aloe vera, C. PDS, D. PDS + Aloe vera; E. PHBV; F. PHBV + Aloe vera. Scales (A-F): 60 µm. Scales in the insert image (A-F): 6 µm.
Figure 3 shows the immunofluorescence images of rat dorsal root ganglion (DRG) explant neurons cultured in the presence of aligned synthetic PHBV polymer nanofibers (Figure 3A), and in the presence of the aligned hybrid PHBV and Aloe vera nanofibers (Figure 3B) of the invention. Figure 3C is a representative diagram of Figures 3A and 3B, showing the neuronal bodies (spherical structures) and their nerve projections following the path of the aligned nanofibers. The arrows in Figures 3A, 3B, and 3C indicate the location of the growth cones. Scale (Figures 3A and 3B): 200 µm.
Figure 4 shows the scanning electron microscopy micrograph of unaligned nanofibers oriented in different directions with respect to one another: A. Honey + PHVB; B. Aloe vera + PHBV, and C. PHBV. Scale (Figures 4A, 4B, and 4C): 20 µm.
Figure 5A shows an immunofluorescence image (confocal microscope) of rat DRG explant neurons cultured in the presence of unaligned nanofibers of Figure 4A. Figure 5B is a representative diagram of Figure 5A showing the neuronal bodies (spherical central structure) and the growth of their neurites following the path of the unaligned hybrid nanofibers.

### Detailed Description of the Invention

### Hybrid Aloe vera and synthetic polymer nanofibers

The present invention relates to hybrid nanofibers comprising a mixture of
i) the components of an Aloe vera gel and
ii) a synthetic polymer,
   wherein the synthetic polymer is selected from poly-3-hydroxybutyrate-co-3-hydroxyvalerate (PHBV), poly-L-lactic acid (PLLA), polydioxanone (PDS), and derived mixtures,
   wherein the average diameter of the hybrid nanofibers is comprised between 0.3 and 1.5 microns, and
   wherein the components of an Aloe vera gel are all the components of said Aloe vera gel or Aloe vera gel derivatives selected from gel lyophilisate, gel secretion, plant extract, solution of the gel, or in solution without aloin and/or with a high concentration of acemannan.

In the context of the present invention, hybrid Aloe vera nanofibers refer to hybrid nanofibers comprising a mixture of the components of the Aloe vera gel and a synthetic polymer selected from poly-3-hydroxybutyrate-co-3-hydroxyvalerate (PHBV), poly-L-lactic acid (PLLA), polydioxanone (PDS), and derived mixtures.

The Aloe vera gel is extracted from the Aloe vera plant of the Liliaceae family. The Aloe vera plant is a succulent plant containing more than 75 potentially bioactive components such as vitamins, enzymes, minerals, sugars, saponins, salicylic acids, and amino acids, including among such components the essential amino acids lysine, threonine, valine, leucine, phenylalanine, and methionine. Furthermore, it contains anthrones (aloe-emodin, aloin A, aloin B, 8-O-methyl-7-hydroxyaloin A, 8-O-methyl-7-hydroxyaloin B, and 10-hydroxyaloin A), phenyl pyrones (aloin A and aloin B), and chromones (aloesin, 8-C-glucosyl-7-O-methyl-(S)-aloesol, isoaloeresin D, and aloeresin E). The plant has spear-shaped leaves containing Aloe vera gel which provides rigidity to the leaves. The Aloe vera plant is a tropical or subtropical plant of the genus Aloe that usually requires temperatures above 10°C for cultivation. In a preferred embodiment, the Aloe vera gel of the hybrid Aloe vera nanofibers of the invention comes from Aloe vera plants of the Canary Islands. In another particular embodiment, the Aloe vera gel of the hybrid nanofibers of the invention comes from the *Aloe barbadensis Miller* plant

Aloe vera gel is usually isolated from the plant by means of methods known in the state of the art. In that sense, patent document US 2016/0015041 A1, for example, discloses a method based on slicing Aloe vera leaves and grinding them to extract the gel. Other methods for gel extraction are disclosed in patent documents US 3,878,197 or US 4959214 A.

In the context of the invention, the term "the components of an Aloe vera gel" refers to the components present in any Aloe vera gel extracted from the Aloe vera plant and, where appropriate, sterilized and stabilized; said components including, among others, mucilaginous polysaccharides bound to sugars such as glucose, acemannan, mannose, glucomannose, rhamnose, xylose, arabinose, galactose, aldopentose, and cellulose; carbohydrates, acids, organic salts, enzymes, sterols, triacylglycerides, amino acids, RNA, traces of alkaloids, vitamins, and various minerals. Some documents of the state of the art disclosing the analysis of the composition of Aloe vera gels are Reynolds et al. (Journal of Ethnopharmacology 68 (1999) 3-37) and J. H. Hamman (Molecules, 13 (2008)1599-1616).

Aloe vera gel extracted from the plant oxidizes rapidly in the open air, breaking down and often losing its properties. For use in a location far away from where it originated, the Aloe vera gel must be sterilized and stabilized once isolated from the plant. Several new technologies such as preservation by means of high hydrostatic pressures, ohmic heating, electric pulses, microwaves, gamma radiation, and ultrasound have been described for Aloe vera gel stabilization. R.N. Dominguez-Fernández (Revista Mexicana de Ingeniería Química 11 (2012) 23-43) mentions some of the methods known in the state of the art for Aloe vera gel stabilization.

The authors have observed that the hybrid Aloe vera nanofibers of the invention are also biodegradable and biocompatible. It is believed that these properties are due to the presence of the components of the Aloe vera gel in the hybrid nanofibers.

The hybrid nanofibers of the invention comprising a mixture of the components of the Aloe vera gel and a synthetic polymer selected from poly-3-hydroxybutyrate-co-3-hydroxyvalerate (PHBV), poly-L-lactic acid (PLLA), polydioxanone (PDS), and derived mixtures have an average diameter comprised between 0.3 and 1.5 microns, preferably between 0.5 and 1.5 microns, more preferably between 0.8 and 1.5 microns. In a preferred embodiment, the hybrid nanofibers have an average diameter comprised between 0.8 and 1.3 microns, preferably between 0.9 and 1.2 microns, more preferably between 1 and 1.1 microns. The diameters of the hybrid nanofibers disclosed in the present application were measured from SEM images using the Image J computer program (NIH, USA). Average diameters were calculated considering 100 fibers of each sample. The inventors have observed that by having the indicated diameter ranges, the hybrid Aloe vera nanofibers of the invention act as a support for the adherence and guidance of nerve cells in axonal growth during a process of regeneration and healing of any tissue or organ having nerve structures. In this sense, the hybrid nanofibers of the present invention are particularly useful as a support for axonal regeneration in the peripheral nervous system (PNS) and the central nervous system (CNS). Furthermore, the inventors of the present invention have observed that the hybrid Aloe vera nanofibers of the invention allow for the growth of longer regenerated neurites than in the presence of synthetic polymer nanofibers, with the same time of action, and without requiring growth factor supplements.

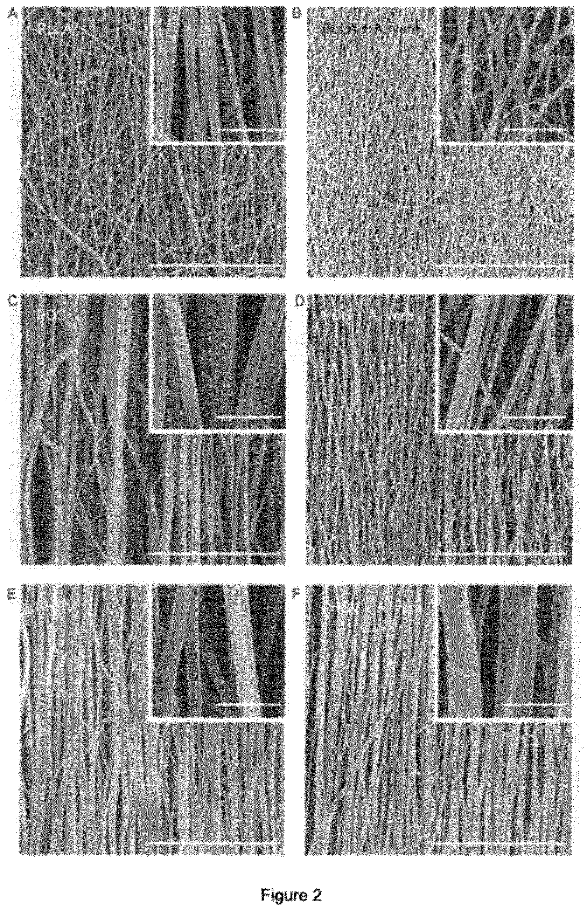

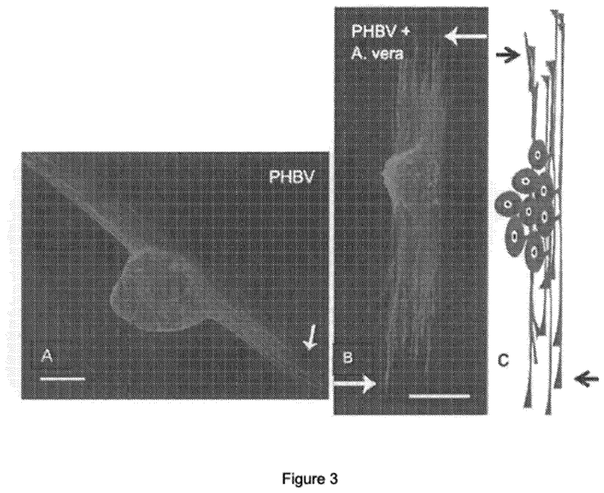

On the other hand, the inventors have found that the presence of the components of an Aloe vera gel in the hybrid nanofibers can, in some cases, reduce the diameter of the synthetic polymer nanofibers prepared under the same conditions. In that sense, hybrid Aloe vera/PDS nanofibers have a smaller diameter than PDS nanofibers prepared under the same conditions, as shown in Figures 2C and 2D. This very effect has also been observed in hybrid Aloe vera/PLLA nanofibers (Figures 2A and 2B). In contrast, the diameter of Aloe vera/PHBV nanofibers barely changes with respect to the diameter of PHBV nanofibers. Wang et al. (Acta Biomater., 2010, 6(8) 2970-2978) discloses that the diameter of synthetic PLLA nanofibers influences nerve growth. The inventors of the present invention have observed that hybrid PHBV/Aloe vera nanofibers improve nerve growth with respect to pure PHBV nanofibers having the same diameter. Figure 3 shows the growth of nerve endings in the presence of aligned hybrid Aloe vera/PHBV nanofibers of the invention (Figure 3A) and in the presence of aligned synthetic PHBV polymer nanofibers (Figure 3B). The images show that the growth of the nerve endings is surprisingly greater in the presence of the hybrid nanofibers of the invention.

In a particular embodiment, the hybrid nanofibers of the invention contain between 3 and 5% by weight of the components of an Aloe vera gel and between 10 and 12% by weight of synthetic polymer, wherein the synthetic polymer is selected from poly-3-hydroxybutyrate-co-3-hydroxyvalerate (PHBV), poly-L-lactic acid (PLLA), polydioxanone (PDS), and derived mixtures, and have an average diameter of the hybrid nanofibers comprised between 0.3 and 1.5 microns. The hybrid nanofibers preferably contain 4% by weight of the components of an Aloe vera gel and 11% by weight of synthetic polymer.

In a particular embodiment, the hybrid nanofibers of the invention contain between 3 and 5% by weight of the components of an Aloe vera gel and between 10 and 12% by weight of a synthetic polymer, wherein the synthetic polymer is selected from poly-3-hydroxybutyrate-co-3-hydroxyvalerate (PHBV), poly-L-lactic acid (PLLA), polydioxanone (PDS), and derived mixtures, and wherein the hybrid nanofibers have an average diameter comprised between 0.8 and 1.5 microns. The hybrid nanofibers preferably contain 4% by weight of the components of an Aloe vera gel and 11% by weight of synthetic polymer.

In a particular embodiment, the ratio by weight of the components of an Aloe vera gel and synthetic polymer in the hybrid nanofibers of the invention is comprised between 17:83 and 33:67. The ratio by weight of the components of an Aloe vera gel/synthetic polymer is preferably comprised between 20:80 and 30:70, more preferably between 23:77 and 27:73. In a preferred embodiment, the ratio by weight of the components of an Aloe vera gel/synthetic polymer is comprised between 24:76 and 26:74.

In another embodiment, the hybrid nanofibers of the invention can be aligned, i.e., oriented in one and the same direction, or disorganized, i.e., oriented in different directions with respect to one another. The hybrid nanofibers of the invention are preferably aligned. The authors of the present invention have observed that when the hybrid nanofibers of the invention are aligned, greater nerve growth occurs.

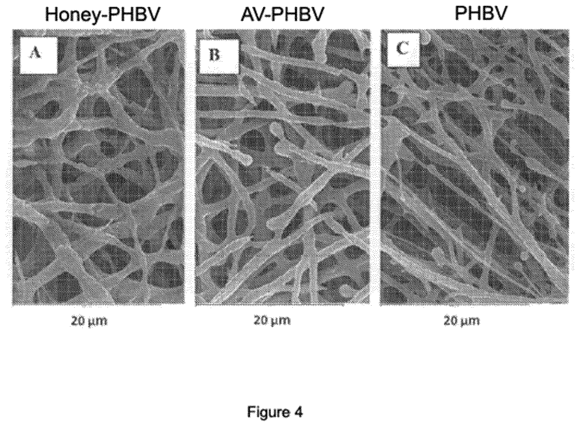

In another particular embodiment, the hybrid nanofibers of the invention are oriented in different directions with respect to one another. Figure 4B shows the scanning electron microscopy image of the hybrid Aloe vera/PHBV nanofibers of the invention oriented in different directions with respect to one another.

The *in vitro* experiments conducted have demonstrated that nanofibers oriented in different directions with respect to one another act as a guide for the migration of glial cells (Schwann cells), fibroblasts, and as a guide for growing neuronal axons. In particular, when oriented in different directions with respect to one another, the hybrid nanofibers of the invention mimic the unaligned extracellular matrix of organs such as the skin (dermis), whereas when oriented in the same direction, they mimic the aligned extracellular matrix of structures such as nerves. The hybrid nanofibers of the invention are particularly useful as a support for the regeneration of different tissues/organs.

In a particular embodiment, the hybrid nanofibers of the invention comprise a mixture of the components of an Aloe vera gel obtained from the *Aloe barbadensis Miller* plant and poly-3-hydroxybutyrate-co-3-hydroxyvalerate (PHBV), wherein said hybrid nanofibers have an average diameter between 0.8 and 1.5 microns, and wherein the nanofibers are aligned in a specific direction or oriented in different directions with respect to one another.

In another particular embodiment, the hybrid nanofibers comprise a mixture of the components of an Aloe vera gel obtained from the *Aloe barbadensis Miller* plant and poly-L-lactic acid (PLLA), wherein the hybrid nanofibers have an average diameter between 0.8 and 1.5 microns and are aligned in a specific direction or oriented in different directions with respect to one another.

In another particular embodiment, the hybrid nanofibers comprise a mixture of the components of an Aloe vera gel obtained from the *Aloe barbadensis Miller* plant and polydioxanone (PDS), wherein the hybrid nanofibers have an average diameter between 0.8 and 1.5 microns and are aligned in a specific direction or oriented in different directions with respect to one another.

### Method of producing hybrid Aloe vera and synthetic polymer nanofibers

In one aspect, the invention relates to the method for producing hybrid nanofibers, wherein the method comprises:
a) preparing a solution of an Aloe vera gel or of Aloe vera gel derivatives in a solvent selected from hexafluoride-2-propanol, polyvinyl alcohol (PVA), a chloroform:methanol solution, or derived mixtures,
b) mixing the solution of Aloe vera gel or of Aloe vera gel derivatives of step a) with a synthetic polymer selected from poly-3-hydroxybutyrate-co-3-hydroxyvalerate (PHBV), poly-L-lactic acid (PLLA), polydioxanone (PDS), and derived mixtures to form a hybrid polymer solution, and
c) injecting the mixture of step b) into electrospinning equipment for producing hybrid Aloe vera nanofibers by electrospinning, wherein the Aloe vera gel derivatives in step a) are selected from gel lyophilisate, gel secretion, plant extract, solution of the gel, or in solution without aloin and/or with a high concentration of acemannan.

According to the method described above, a solution of an Aloe vera gel or Aloe vera gel derivatives in hexafluoride-2-propanol (HFIP), polyvinyl alcohol (PVA), a chloroform:methanol solution, or derived mixtures, is prepared in step a). Said solution usually has a concentration of 25 to 50 mg/ml of Aloe vera gel. In a particular embodiment, the solvent of the solution of Aloe vera is a chloroform:methanol solution with a volume ratio of 3:1.

The Aloe vera gel used in the present invention can be of different origins. Nevertheless, the Aloe vera gel from Canary Islands is preferably used. In a particular embodiment, the Aloe vera gel of the hybrid nanofibers of the invention comes from the *Aloe barbadensis Miller* plant.

In the context of the present invention, the term "Aloe vera gel derivatives" refers to the different forms in which the Aloe vera gel may be presented, such as gel lyophilisate, gel secretion, plant extract, solution of the gel, or in solution without aloin, and/or with a high concentration of acemannan. In a particular embodiment, the Aloe vera gel derivatives in step a) are selected from gel lyophilisate, gel secretion, plant extract, solution of the gel, in solution without aloin, and/or with a high concentration of acemannan.

The so-called "components of an Aloe vera gel" are present in all Aloe vera gel derivatives.

In step b) of the method described above, the solution of the Aloe vera gel or of Aloe vera gel derivatives of step a) is mixed with a synthetic polymer selected from poly-3-hydroxybutyrate-co-3-hydroxyvalerate (PHBV), poly-L-lactic acid (PLLA), polydioxanone (PDS), and derived mixtures to form a hybrid polymer solution.

In a particular embodiment, the hybrid polymer solution contains Aloe vera gel obtained from the *Aloe barbadensis Miller* plant, and the synthetic polymer is poly-3-hydroxybutyrate-co-3-hydroxyvalerate (PHBV).

In a preferred embodiment, the ratio by weight of the Aloe vera gel and synthetic polymer in the hybrid polymer solution of step b) is comprised between 17:83 and 33:67. The ratio by weight of the Aloe vera gel and synthetic polymer influences the average diameter of the hybrid nanofibers. Generally, the diameter of the nanofibers is greater when the concentration of the synthetic polymer in the solution increases. The ratio by weight of the Aloe vera gel/synthetic polymer in the hybrid polymer solution of step b) is preferably comprised between 20:80 and 30:70, more preferably between 23:77 and 27:73. In a preferred embodiment, the ratio by weight of the Aloe vera gel/synthetic polymer is comprised between 24:76 and 26:74.

In step c) of the method of the invention, the mixture of step b) is injected into electrospinning equipment for producing hybrid Aloe vera nanofibers by electrospinning. The electrospinning process is generally affected by system parameters such as the molecular weight of the polymer, molecular weight distribution, and dissolution properties such as viscosity, and surface tension. Furthermore, the electrospinning process can be affected by process parameters such as the flow rate, the electric potential, the distance between the capillary and the collector, etc. These parameters are optimized for controlling the characteristics of the nanofibers that are obtained.

In the electrospinning equipment, the mixture is loaded into a syringe pump connected to an electrode. The solution is driven at a flow rate between 0.7 and 1.2 ml/h, preferably at a flow rate between 0.8 and 1 ml/h, more preferably between 0.9 and 1 ml/h; and applying a potential of 10 to 15 kV, preferably 11 to 14 kV, more preferably 12 to 13 kV, from the syringe to a rotating wheel collector. The solution can also be driven at other flow speeds. For example, the solution can be driven at a flow rate between 2 and 3 ml/h, preferably between 2.5 and 2.8 ml/h, more preferably at 2.75 ml/h. Furthermore, other potentials, such as 9 KV, for example, can also be applied. In a particular embodiment, the solution is driven at 2.75 ml/h and applying a potential of 9 KV.

The rotary wheel collector can rotate at a speed between 2000 and 4000 rpm. In a particular embodiment, the rotating speed of the wheel collector is comprised between 3000 and 3500 rpm. In particular, when the rotating speed is comprised between 3000 and 3500 rpm, the hybrid nanofibers obtained are aligned. The wheel collector can likewise rotate at other speeds. In particular, when the rotating speed is comprised between 100 and 300 rpm, preferably 200 rpm, the nanofibers obtained are not aligned and are oriented in different directions with respect to one another.

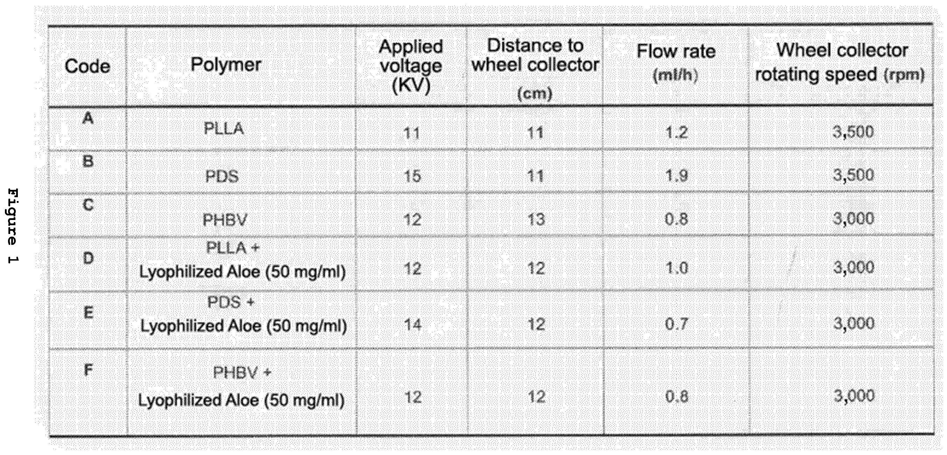

The rotary wheel collector is located at a distance of 8 to 12 cm from the syringe pump, preferably at a distance of 9 to 11 cm from the syringe pump, more preferably at a distance of 10 cm from the syringe pump. The conditions used in electrospinning are environmental conditions with a humidity of 60-70% and a temperature of 20-30°C. Figure 1 of the present application shows, by way of example, the conditions used for producing synthetic polymer nanofibers and for producing the hybrid nanofibers of the invention by electrospinning according to step c) of the method of the invention under environmental conditions of 25±1°C and with a constant relative humidity of 65±5%.

In another additional aspect, the invention relates to hybrid nanofibers comprising a mixture of the components of the Aloe vera gel and a synthetic polymer selected from poly-3-hydroxybutyrate-co-3-hydroxyvalerate (PHBV), poly-L-lactic acid (PLLA), polydioxanone (PDS), and derived mixtures, wherein the hybrid nanofibers have an average diameter comprised between 0.3 and 1.5 microns, obtained by means of the method of the invention described above. Figures 2B, 2D, and 2F show SEM micrographs of the hybrid nanofibers of the invention obtained by means of the described method.

In a particular embodiment, the hybrid nanofibers obtained by means of the described method are aligned in a specific direction or oriented in different directions with respect to one another.

The hybrid Aloe vera nanofibers of the present invention can be used for tissue engineering applications, particularly for nerve tissue regeneration. In this sense, an aspect of the invention relates to the use of the hybrid nanofibers for producing a medicinal product for nerve tissue regeneration in any vertebrate or for promoting the growth, proliferation, or differentiation of any cell type located in any tissue, organ, or organ system where the nerve tissue is present.

In one aspect, the invention relates to the hybrid Aloe vera nanofibers of the invention for use as a medicinal product.

In another aspect, the invention relates to the use of the hybrid Aloe vera nanofibers for producing a medicinal product for nerve tissue regeneration in any vertebrate or for promoting the growth, proliferation, or differentiation of any cell type located in any tissue, organ, or organ system where the nerve tissue is present.

In another aspect, the invention relates to the hybrid Aloe vera nanofibers of the invention for use in the treatment for nerve tissue regeneration in any vertebrate, or for the growth, proliferation, or differentiation of any cell type located in any tissue, organ, or organ system where the nerve tissue is present.

In a particular embodiment, the hybrid Aloe vera nanofibers of the invention are used for producing a medicinal product for nerve tissue regeneration in any vertebrate or for promoting the growth, proliferation, or differentiation of any cell type located in any tissue, organ, or organ system where the nerve tissue is present, in the absence of any other additive or growth factor which promotes nerve tissue regeneration.

In particular, the hybrid Aloe vera nanofibers of the invention can be used for nerve tissue regeneration in nerve connection prostheses, dressings, sutures in skin wounds, and surgical meshes. In this sense, an aspect of the invention relates to a tubular prosthesis comprising the hybrid Aloe vera nanofibers of the invention. Another aspect of the present invention relates to dressings comprising the hybrid Aloe vera nanofibers of the invention. Another additional aspect relates to sutures comprising the hybrid nanofibers of the invention. Furthermore, another aspect relates to surgical meshes comprising the hybrid nanofibers of the invention

Likewise, the hybrid Aloe vera nanofibers of the invention can also be used for regenerating other tissues, such as tendon, ligament and bone tissues.

### Examples

### 1. Preparing aligned hybrid Aloe vera and synthetic polymer fibers

First, a solution of 25-50 mg/ml of Aloe vera (Prod. No. 001, Laboratories Luciano Reverón e hijos S.L., Tenerife) in hexafluoro-2-propanol (HFIP) was prepared. To obtain the hybrid solution (AV/PHBV), 10-12% (w/w) of PHBV (Sigma-Aldrich, Prod. No. 403121) was added to the preceding solution. To obtain aligned nanofibers by means of the electrospinning technique, the hybrid polymer solution was loaded into a syringe pump (Hardvard Apparatus PhD Ultra) with a needle (G20, 0.9 mm in diameter) the tip of which was connected to an electrode (spinnerette). 12-15 KV were applied with a high-voltage source (Spellman 60N300) while the syringe pump discharged the solution (flow rate of 0.9 ml/h) under environmental conditions with a humidity of 60-65% and a temperature of 22-25°C towards a target wheel (90 mm in diameter and 12 mm thick) located 12 cm form the electrode. Bundles of aligned nanofibers were collected on the target wheel rotating at 3000 rpm. Figure 2F shows SEM micrographs of the nanofibers that were obtained.

Additionally, aligned hybrid fibers of the invention consisting of PLLA and Aloe vera, PDS and Aloe vera and PHVB and Aloe vera, and synthetic PLLA, PDS, and PHBV polymer fibers were prepared following the same method. Figures 2A to 2E show SEM micrographs of the aligned fibers obtained from the synthetic PLLA polymer (Figure 2A), PDS polymer (Figure 2C), and PHBV polymer (Figure 2E); as well as from the hybrid Aloe vera fibers of the invention consisting of PLLA + Aloe vera (Figure 2B), PDS + Aloe vera (Figure 2D) following the same method that has been described.

### 2. Neuritic growth comparative assays

Comparative assays were performed using newborn rat (Sprague Dawley) dorsal root ganglion (DRG) explant culture in a) a standard culture medium [DMEM/F12 (1:1)] to establish the control conditions, and b) in DMEM/F12 (1:1) containing the invented hybrid nanometric matrix (AV PHBV) and other pure reference substances (PHBV and PLLA) as the experimental substrate for neuritic growth. The rat DRG explants were incubated in an oven at 37°C and under a 5% CO₂ atmosphere for 6 days, and the culture medium was changed after 3 days.

After the incubation period has elapsed, the rat DRG explants were fixed with a 4% paraformaldehyde solution in phosphate-buffered saline and immunolabeled with antibodies specific for neuron identification. Digital images were then taken in a fluorescence microscope equipped with an image capturing system. The images were processed for statistical analysis.

A statistical analysis was performed in each of the polymers, the positive area ratios being summarized as medians and interquartile ranges in each of the treatment groups. The statistical analyses demonstrated that the highest neuritic growth rate occurred with the aligned hybrid AV/PHBV nanofibers in comparison with the absence of aloe in aligned pure PHBV nanofibers (p<0.001) and pure PLLA nanofibers (p=0.049).

### 3. Preparing hybrid fibers oriented in different directions with respect to one another

First, a solution of 50 mg/ml of Aloe vera (Prod. No. 001 , Laboratories Luciano Reverón e hijos S.L , Tenerife) in hexafluoro-2-propanol (HFIP) was prepared. The Aloe vera of the solution was lyophilized and filtered Aloe vera having a size of 22 µm. To obtain the hybrid solution (AV/PHBV), 10% by weight of PHBV (Sigma-Aldrich, Prod. No. 403121) was added to the preceding solution (400 mg of PHBV in 3,600 mg HFIP+ Aloe vera).

To obtain the nanofibers by means of the electrospinning technique, the hybrid polymer solution was loaded into a syringe pump (Hardvard Apparatus PhD Ultra) with a needle (16G) the tip of which was connected to an electrode (spinnerette). 9 KV were applied with a high-voltage source (Spellman 60N300) while the syringe pump discharged the solution (flow rate of 2.75 ml/h) under environmental conditions with a humidity of 60-65% and a temperature of 25-26°C towards a target wheel (90 mm in diameter/8 mm thick) located 10 cm from the electrode. Unaligned nanofibers oriented in different directions with respect to one another were collected on the target wheel rotating at 200 rpm. Figure 4B shows SEM micrographs of the hybrid Aloe vera nanofibers that were obtained. The diameters of the obtained nanofibers were measured from the SEM images using the Image J computer program (NIH, USA). The average measured diameter of the hybrid Aloe vera nanofibers obtained is 1.0241 µm, the diameter of the unaligned hybrid nanofibers that were obtained ranging between 0.9854 µm and 1.0628 µm.

The same method was followed to manufacture unaligned hybrid honey fibers oriented in different directions with respect to one another, but using honey from a local beekeeper in Gran Canaria who is registered in the General Health Registry for Food Companies and Foodstuffs (RGSEAA): Maria del Rosario Cazorla Lopez, RGSEAA No.: 23.03229/GC, instead of Aloe vera. Figure 4A shows SEM micrographs of the hybrid honey and PHBV nanofibers that were obtained. The diameters of the obtained nanofibers were measured from the SEM images using the Image J computer program (NIH, USA). The average measured diameter of the obtained hybrid honey nanofibers is 1.2528 µm, the diameter of the unaligned hybrid nanofibers that were obtained ranging between 0.9801 µm and 1.5255 µm.

Unaligned PHVB nanofibers were prepared for comparison. In this case, 12% by weight of PHBV (Sigma-Aldrich, Prod. No. 403121) in hexafluoro-2-propanol (HFIP) was added to obtain a solution with a concentration of 10% by weight of PHBV. The hybrid polymer solution was loaded into a syringe pump (Hardvard Apparatus PhD Ultra) with a needle (16G) the tip of which was connected to an electrode (spinnerette). 12 KV were applied with a high-voltage source (Spellman 60N300) while the syringe pump discharged the solution (flow rate of 1 ml/h) under environmental conditions with a humidity of 60-65% and a temperature of 25-26°C towards a target wheel (90 mm in diameter/8 mm thick) located 12 cm from the electrode. Nanofibers oriented in different directions with respect to one another were collected on the target wheel rotating at 200 rpm. Figure 4C shows SEM micrographs of the nanofibers that were obtained. The diameters of the obtained nanofibers were measured from the SEM images using the Image J computer program (NIH, USA). The average measured diameter of the unaligned PHVB nanofibers is 0.9130 µm, the diameter of the unaligned PHVB nanofibers obtained ranging between 0.7803 µm and 1.0457 µm.

### 4. In vitro neuritic growth comparative assays

Comparative assays were performed using newborn rat (Sprague Dawley) dorsal root ganglion (DRG) explant culture in a standard culture medium [DMEM/F12 (1:1)], the nanometric matrix containing the unaligned honey/PHBV, AV/PHBV, and PHBV fibers of Example 3 as an experimental substrate for neuritic growth. The rat DRG explants were incubated in an oven at 37°C and under a 5% CO₂ atmosphere for 6 days, and the culture medium was changed after 3 days.

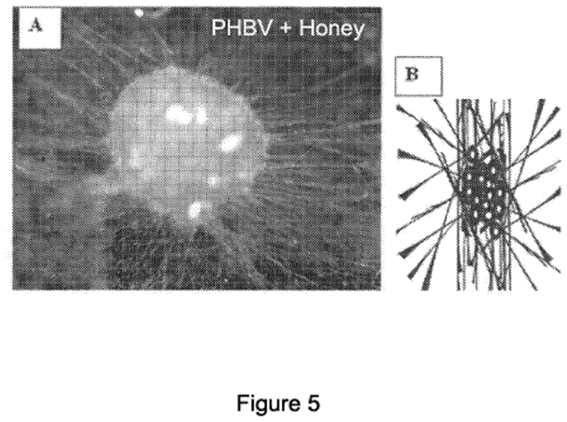

After the incubation period has elapsed, the rat DRG explants were fixed with a 4% paraformaldehyde solution in phosphate-buffered saline and immunolabeled with specific antibodies for the identification of neurons, Schwann cells, and cell nuclei. Digital images were then taken in a fluorescence microscope equipped with an image capturing system. Greater neuritic growth was observed in the presence of hybrid honey/PHBV and AV/PHBV nanofibers than in the presence of PHBV nanofibers. Figure 5A shows the immunofluorescence image of the growth of many neurites in all directions from the rat DRG explant (spherical central structure in the image). The growing neurites follow the path of the unaligned hybrid nanofibers, as depicted in the diagram of Figure 5B.

### 5. In vivo skin wound healing comparative assays

With the required authorization from the Ethics Committee on Animal Experimentation, the murine model for wound (8 mm in diameter) repair (subcutaneous ring) was used to mimic healing by second intention in humans (chronic skin ulcers). A comparative study was performed on the effect of the nanometric matrix containing the unaligned hybrid honey/PHBV, AV/PHBV, and PHBV nanofibers of Example 3 with daily treatments with natural honey and commercial dressing (Mepilex border). The mentioned matrices were applied on the wound bed with no additional care other than protecting same with a surgical adhesive. After 8 days, samples were taken for microbiological cultures (*Staphylococcus aureus* and *E. coli*). The preliminary data indicates that wound closure occurred earlier in groups treated with the hybrid matrices: honey/PHBV (12.5 days on average), AV/PHBV (13 days on average) and daily treatments with natural honey (13.3 days on average) compared to groups treated with pure PHBV matrix (14.3 days on average) or the commercial dressing (15 days on average). The bacteriological analyses were negative.

## Claims

1. Hybrid nanofibers comprising a mixture of:
i) the components of an Aloe vera gel, and
ii) a synthetic polymer,
wherein the synthetic polymer is selected from poly-3-hydroxybutyrate-co-3-hydroxyvalerate (PHBV), poly-L-lactic acid (PLLA), polydioxanone (PDS), and derived mixtures,
wherein the average diameter of the hybrid nanofibers is comprised between 0.3 and 1.5 microns; and
wherein the components of an Aloe vera gel are all the components of said Aloe vera gel or Aloe vera gel derivatives selected from gel lyophilisate, gel secretion, plant extract, solution of the gel, or in solution without aloin and/or with a high concentration of acemannan.

2. Hybrid nanofibers according to claim 1, wherein the hybrid nanofibers have an average diameter comprised between 0.8 and 1.5 microns.

3. Hybrid nanofibers according to any of claims 1 to 2, wherein the hybrid nanofibers comprise between 3 and 5% by weight of the components of the Aloe vera, and between 10 and 12% by weight of synthetic polymer.

4. Hybrid nanofibers according to any of claims 1 to 3, wherein the Aloe vera gel is obtained from the Aloe barbadensis Miller plant.

5. Hybrid nanofibers according to any of claims 1 to 4, wherein said nanofibers are aligned in a specific direction or oriented in different directions with respect to one another.

6. Hybrid nanofibers according to claim 1, wherein said nanofibers comprise a mixture of:
i) the components of an Aloe vera gel obtained from the Aloe barbadensis Miller plant, and
ii) poly-3-hydroxybutyrate-co-3-hydroxyvalerate (PHBV);
wherein said hybrid nanofibers have an average diameter between 0.8 and 1.5 microns, and
wherein the nanofibers are aligned in a specific direction or oriented in different directions with respect to one another.

7. A method for producing hybrid nanofibers according to claims 1 to 6, wherein the method comprises:
a) preparing a solution of an Aloe vera gel or of Aloe vera gel derivatives in a solvent selected from hexafluoride-2-propanol, polyvinyl alcohol (PVA), a chloroform:methanol solution, or derived mixtures,
b) mixing the solution of Aloe vera gel or of Aloe vera gel derivatives of step a) with a synthetic polymer selected from poly-3-hydroxybutyrate-co-3-hydroxyvalerate (PHBV), poly-L-lactic acid (PLLA), polydioxanone (PDS), and derived mixtures to form a hybrid polymer solution, and
c) injecting the mixture of step b) into electrospinning equipment for producing hybrid Aloe vera nanofibers by electrospinning; wherein the Aloe vera gel derivatives in step a) are selected from gel lyophilisate, gel secretion, plant extract, solution of the gel, or in solution without aloin and/or with a high concentration of acemannan.

8. The method according to claim 7, wherein in step b) the ratio by weight of the Aloe vera gel and the synthetic polymer is comprised between 17:83 and 33:67.

9. The method according to any of claims 7 or 8, wherein the Aloe vera gel is obtained from the Aloe barbadensis Miller plant and the synthetic polymer is poly-3-hydroxybutyrate-co-3-hydroxyvalerate (PHBV).

10. Hybrid nanofibers according to any of claims 1 to 6 for use as a medicinal product.

11. Use of the hybrid nanofibers according to any of claims 1 to 6 for producing a medicinal product for nerve tissue regeneration in any vertebrate or for promoting the growth, proliferation, or differentiation of any cell type located in any tissue, organ, or organ system where the nerve tissue is present.

12. Use of the hybrid nanofibers according to any of claims 1 to 6 for producing tubular prostheses, dressings, sutures, and surgical meshes.

13. Tubular prosthesis, dressing, sutures or surgical meshes comprising the hybrid nanofibers according to claims 1 to 6.

## Patentansprüche

1. Hybride Nanofasern, die eine Mischung umfassen aus:
i) den Bestandteilen eines Aloe-Vera-Gels, und
ii) einem synthetischen Polymer,
wobei das synthetische Polymer ausgewählt ist aus Poly-3-hydroxybutyrat-co-3-hydroxyvalerat (PHBV), Poly-L-Milchsäure (PLLA), Polydioxanon (PDS) und abgeleiteten Mischungen,
wobei der durchschnittliche Durchmesser der Hybrid-Nanofasern zwischen 0,3 und 1,5 Mikrometern liegt; und
wobei die Komponenten eines Aloe-Vera-Gels alle Komponenten des Aloe-Vera-Gels oder Aloe-Vera-Gel-Derivate sind, ausgewählt aus Gel-Lyophilisat, Gel-Sekret, Pflanzenextrakt, Lösung des Gels oder in Lösung ohne Aloin und/oder mit einer hohen Konzentration an Acemannan.

2. Hybride Nanofasern nach Anspruch 1, wobei die hybriden Nanofasern einen durchschnittlichen Durchmesser zwischen 0,8 und 1,5 Mikrometern aufweisen.

3. Hybride Nanofasern nach einem der Ansprüche 1 bis 2, wobei die hybriden Nanofasern zwischen 3 und 5 Gew.-% der Bestandteile der Aloe Vera und zwischen 10 und 12 Gew.-% synthetisches Polymer enthalten.

4. Hybride Nanofasern nach einem der Ansprüche 1 bis 3, wobei das Aloe-Vera-Gel aus der Aloe-Barbadensis-Miller-Pflanze gewonnen wird.

5. Hybride Nanofasern nach einem der Ansprüche 1 bis 4, wobei die Nanofasern in einer bestimmten Richtung ausgerichtet oder in verschiedenen Richtungen zueinander orientiert sind.

6. Hybride Nanofasern nach Anspruch 1, wobei die Nanofasern eine Mischung umfassen aus:
i) den Bestandteilen eines Aloe-Vera-Gels, das aus der Aloe-Barbadensis-Miller-Pflanze gewonnen wird, und
ii) Poly-3-hydroxybutyrat-co-3-hydroxyvalerat (PHBV);
wobei die hybriden Nanofasern einen durchschnittlichen Durchmesser zwischen 0,8 und 1,5 Mikrometern haben, und
wobei die Nanofasern in einer bestimmten Richtung ausgerichtet oder in verschiedenen Richtungen zueinander orientiert sind.

7. Verfahren zur Herstellung von hybriden Nanofasern nach einem der Ansprüche 1 bis 6, wobei das Verfahren umfasst:
a) Herstellen einer Lösung eines Aloe-Vera-Gels oder von Aloe-Vera-Gel-Derivaten in einem Lösungsmittel, ausgewählt aus Hexafluorid-2-propanol, Polyvinylalkohol (PVA), einer Chloroform:Methanol-Lösung oder abgeleiteten Mischungen,
b) Mischen der Lösung von Aloe-Vera-Gel oder von Aloe-Vera-Gel-Derivaten aus Schritt a) mit einem synthetischen Polymer, ausgewählt aus Poly-3-hydroxybutyrat-co-3-hydroxyvalerat (PHBV), Poly-L-Milchsäure (PLLA), Polydioxanon (PDS) und abgeleiteten Mischungen, um eine Hybridpolymerlösung zu bilden, und
c) Einspritzen der Mischung aus Schritt b) in eine Elektrospinnanlage zur Herstellung von hybriden Aloe-Vera-Nanofasern durch Elektrospinnen; wobei die Aloe-Vera-Gel-Derivate in Schritt a) ausgewählt sind aus Gel-Lyophilisat, Gel-Sekret, Pflanzenextrakt, Lösung des Gels oder in Lösung ohne Aloin und/oder mit einer hohen Konzentration an Acemannan.

8. Verfahren nach Anspruch 7, wobei in Schritt b) das Gewichtsverhältnis zwischen dem Aloe-Vera-Gel und dem synthetischen Polymer zwischen 17:83 und 33:67 liegt.

9. Verfahren nach einem der Ansprüche 7 oder 8, wobei das Aloe-Vera-Gel aus der Aloe-Barbadensis-Miller-Pflanze gewonnen wird und das synthetische Polymer Poly-3-hydroxybutyrat-co-3-hydroxyvalerat (PHBV) ist.

10. Hybride Nanofasern nach einem der Ansprüche 1 bis 6 zur Verwendung als Arzneimittel.

11. Verwendung der hybriden Nanofasern nach einem der Ansprüche 1 bis 6 zur Herstellung eines Arzneimittels für die Regeneration von Nervengewebe in einem beliebigen Wirbeltier oder zur Förderung des Wachstums, der Proliferation oder der Differenzierung eines beliebigen Zelltyps in einem beliebigen Gewebe, Organ oder Organsystem, in dem das Nervengewebe vorhanden ist.

12. Verwendung der hybriden Nanofasern nach einem der Ansprüche 1 bis 6 zur Herstellung von röhrenförmigen Prothesen, Verbänden, Nahtmaterial und chirurgischen Netzen.

13. Röhrenförmige Prothese, Verband, Nahtmaterial oder chirurgische Netze, die die hybriden Nanofasern nach einem der Ansprüche 1 bis 6 enthalten.

## Revendications

1. Nanofibres hybrides comprenant un mélange :
i) des composants d'un gel d'Aloe vera, et
ii) d'un polymère synthétique,
dans lesquelles le polymère synthétique est choisi parmi le poly-3-hydroxybutyrate-co-3-hydroxyvalérate (PHBV), l'acide poly-L-lactique (PLLA), la polydioxanone (PDS), et des mélanges dérivés,
dans lesquelles le diamètre moyen des nanofibres hybrides est compris entre 0,3 et 1,5 microns ; et
dans lesquelles les composants d'un gel d'Aloe vera sont tous les composants dudit gel d'Aloe vera ou des dérivés de gel d'Aloe vera choisis parmi un lyophilisat de gel, une sécrétion de gel, un extrait de plante, une solution du gel, ou en solution sans aloïne et/ou avec une concentration élevée d'acémannane.

2. Nanofibres hybrides selon la revendication 1, dans lesquelles les nanofibres hybrides ont un diamètre moyen compris entre 0,8 et 1,5 microns.

3. Nanofibres hybrides selon l'une quelconque des revendications 1 à 2, dans lesquelles les nanofibres hybrides comprennent entre 3 et 5 % en poids de composants de l'Aloe vera, et entre 10 et 12 % en poids de polymère synthétique.

4. Nanofibres hybrides selon l'une quelconque des revendications 1 à 3, dans lesquelles le gel d'Aloe vera est obtenu à partir de la plante Aloe barbadensis Miller.

5. Nanofibres hybrides selon l'une quelconque des revendications 1 à 4, dans lesquelles lesdites nanofibres sont alignées dans une direction spécifique ou orientées dans des directions différentes les unes par rapport aux autres.

6. Nanofibres hybrides selon la revendication 1, dans lesquelles lesdites nanofibres comprennent un mélange :
i) des composants d'un gel d'Aloe vera obtenu à partir de la plante Aloe barbadensis Miller, et
ii) de poly-3-hydroxybutyrate-co-3-hydroxyvalérate (PHBV) ;
dans lesquelles lesdites nanofibres hybrides ont un diamètre moyen compris entre 0,8 et 1,5 microns, et
dans lesquelles les nanofibres sont alignées dans une direction spécifique ou orientées dans des directions différentes les unes par rapport aux autres.

7. Procédé de production de nanofibres hybrides selon les revendications 1 à 6, dans lequel le procédé comprend :
a) la préparation d'une solution d'un gel d'Aloe vera ou de dérivés de gel d'Aloe vera dans un solvant choisi parmi l'hexafluorure-2-propanol, l'alcool polyvinylique (PVA), une solution chloroforme:méthanol, ou des mélanges dérivés,
b) le mélange de la solution de gel d'Aloe vera ou de dérivés de gel d'Aloe vera de l'étape a) avec un polymère synthétique choisi parmi le poly-3-hydroxybutyrate-co-3-hydroxyvalérate (PHBV), l'acide poly-L-lactique (PLLA), la polydioxanone (PDS), et des mélanges dérivés pour former une solution de polymère hybride, et
c) l'injection du mélange de l'étape b) dans un équipement d'électrofilage pour produire des nanofibres d'Aloe vera hybrides par électrofilage ;
dans lequel les dérivés de gel d'Aloe vera de l'étape a) sont choisis parmi un lyophilisat de gel, une sécrétion de gel, un extrait de plante, une solution du gel, ou en solution sans aloïne et/ou avec une concentration élevée d'acémannane.

8. Procédé selon la revendication 7, dans lequel dans l'étape b) le ratio en poids du gel d'Aloe vera et du polymère synthétique est compris entre 17:83 et 33:67.

9. Procédé selon l'une quelconque des revendications 7 à 8, dans lequel le gel d'Aloe vera est obtenu à partir de la plante Aloe barbadensis Miller et le polymère synthétique est le poly-3-hydroxybutyrate-co-3-hydroxyvalérate (PHBV).

10. Nanofibres hybrides selon l'une quelconque des revendications 1 à 6 pour une utilisation en tant que médicament.

11. Utilisation des nanofibres hybrides selon l'une quelconque des revendications 1 à 6 pour produire un médicament pour la régénération de tissu nerveux chez tout vertébré ou pour favoriser la croissance, la prolifération ou la différenciation de tout type cellulaire situé dans tout tissu, organe ou système organique où le tissu nerveux est présent.

12. Utilisation des nanofibres hybrides selon l'une quelconque des revendications 1 à 6 pour produire des prothèses tubulaires, des pansements, des sutures, et des treillis chirurgicaux.

13. Prothèse tubulaire, pansement, sutures ou treillis chirurgicaux comprenant les nanofibres hybrides selon les revendications 1 à 6.
